# EUROPÄISCHE PATENTSCHRIFT

(11) **EP 0 589 336 B1**
(45) Veröffentlichungstag und Bekanntmachung des Hinweises auf die Patenterteilung: **08.01.1997**
(21) Anmeldenummer: 93114774.8
(22) Anmeldetag: 14.09.1993
(51) Int. Cl.: C07C 317/48, C07C 317/46, C07C 317/44, C07D 295/14, C07D 211/70, A61K 31/155, A61K 31/54, A61K 31/45, A61K 49/00

(54) **Benzoylguanidine, Verfahren zu ihrer Herstellung, sowie ihre Verwendung als Antiarrhythmika**
Benzoylguanidines, process for their preparation and their use as antiarrhythmic agents
Benzoylguanidines, leur procédé de préparation et leur utilisation comme agents antiarrhythmiques

(30) Priorität: 22.09.1992 DE 4231658; 16.12.1992 DE 4242587
(43) Veröffentlichungstag der Anmeldung: 30.03.1994
(73) Patentinhaber: HOECHST AKTIENGESELLSCHAFT, 65929 Frankfurt am Main (DE)
(72) Erfinder: Lang, Hans-Jochen, Dr., D-65719 Hofheim/Ts. (DE); Weichert, Andreas, Dr., D-60529 Frankfurt am Main (DE); Kleemann, Heinz-Werner, Dr., D-61350 Bad Homburg (DE); Englert, Heinrich, Dr., D-65719 Hofheim/Ts. (DE); Scholz, Wolfgang, Dr., D-65760 Eschborn (DE); Albus, Udo, Dr., D-61197 Florstadt (DE)

(56) Entgegenhaltungen:
- EP-A- 0 416 499
- BIOCHEMICAL AND BIOPHYSICAL RESEARCH COMMUNICATIONS, Bd. 184, Nr. 1 , 15. April 1992, Dulluth, MN, US, Seiten 112 - 117, A. SCHMID et al: "Na+/H+ exchange in porcine cerebral capillary endothelial cells inhibited by a benzoylguanidine derivative"

## Beschreibung

Die Erfindung betrifft ein Benzoylguanidin, ausgewählt aus der Gruppe, bestehend aus 4-Ethylamino-3-methylsulfonylbenzoyl- guanidin-hydrochlorid, 4-N,N-Diethylamino-3-methylsulfonylbenzoyl-guanidin- hydrochlorid, 4-(4-Chlorphenoxy)-3-methylsulfonylbenzoyl-guanidin-hydrochlorid, 4-(3-Chlor-4-fluorphenoxy)-3-methylsulfonylbenzoyl-guanidin-hydrochlorid, 4-(4-Fluorphenoxy)-3-methylsulfonylbenzoyl-guanidin-hydrochlorid, 4-(4-Fluoranilino)-3-methylsulfonylbenzoyl-guanidin-hydrochlorid, 4-(3-Chlor-4-fluoranilino)-3-methylsulfonylbenzoyl-guanidin-hydrochlorid, 4-Isopropyl-3-methylsulfonylbenzoyl-guanidin-hydrochlorid, 4-Isopropyl-3-methylsulfonylbenzoyl-guanidin-methansulfonat, 4-Phenoxy-3-methylsulfonylbenzoyl-guanidin-hydrochlorid, 3-Methylsulfonyl-4-(2,6-cis-dimethylpiperidino)benzoyl-guanidin-methansulfonat, 4-(1-Methylpropyl)-3-methylsulfonylbenzoyl-guanidin-hydrochlorid, 4-Butyl-3-methylsulfonylbenzoyl-guanidin-hydrochlorid, 4-(2-Methylpropyl)-3-methylsulfonylbenzoyl-guanidin-hydrochlorid und 4-(4-Hydroxyphenoxy)-3-methylsulfonylbenzoyl-guanidin-hydrochlorid.

Bevorzugt ist eine Verbindung, ausgewählt aus der Gruppe bestehend aus 4-Isopropyl-3-methylsulfonylbenzoyl-guanidin-methansulfonat, 4-Isopropyl-3-methylsulfonylbenzoyl-guanidin-hydrochlorid, 4-Phenoxy-3-methylsulfonylbenzoyl-guanidin-hydrochlorid und 4-(4-Chlorphenoxy)-3-methylsulfonylbenzoyl-guanidin-hydrochlorid.

Ganz besonders bevorzugt ist 4-Isopropyl-3-methylsulfonylbenzoyl-guanidin-methansulfonat.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 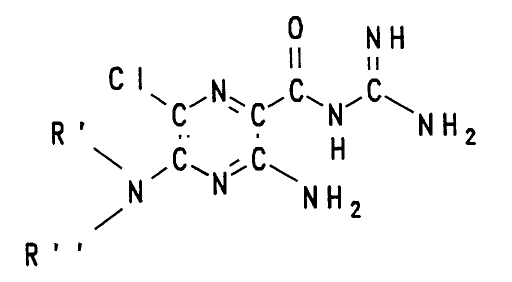 Amilorid: R', R'' = H
Dimethylamilorid: R', R'' = CH₃
Ethylisopropylamilorid: R' = C₂H₅, R'' = CH(CH₃)₂

Darüberhinaus sind Untersuchungen bekannt geworden, die auf antiarrhythmische Eigenschaften von Amilorid hinweisen (Circulation 79; 1257 bis 1263 (1989)). Einer breiten Anwendung als Antiarrhythmikum steht jedoch entgegen, daß dieser Effekt nur schwach ausgeprägt ist und von einer blutdrucksendenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl. 1): 167 (1988) (book of abstracts)). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der Europäischen Offenlegungsschrift 416 499 werden Benzoylguanidine mit antiarrhythmischen Eigenschaften beschrieben.

In der US-Patentschrift 3,780,027 werden Acylguanidine beschrieben, die strukturell den Verbindungen der Formel I ähnlich sind. Der entscheidende Unterschied zu den Verbindungen I besteht darin, daß es sich um trisubstituierte Benzoylguanidine handelt, die sich in ihrem Substitutionsmuster von im Handel befindlichen Diuretika, wie Bumetanid und Furosemid, ableiten und eine für die angestrebte salidiuretische Wirkung wichtige Aminogruppe in Position 2 bzw. 3 zur Carbonylguanidingruppe tragen. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch schäme ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindung auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und - hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Die Erfindung betrifft weiterhin ein Verfahren zur Herstellung der Verbindungen I, dadurch gekennzeichnet, daß man Verbindungen der Formel II 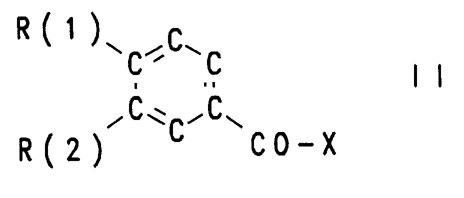 worin R(1) und R(2) die dem im Endprodukt entsprechende Bedeutung hat, und worin X für eine leicht nucleophil substituierbare leaving group steht,
mit Guanidin umsetzt.

Die aktivierten Säurederivate der Formel II, worin X eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, X = Cl), die man ihrerseits wiederum in an sich bekannter Weise aus den zugrundeliegenden Carbonsäuren (Formel II, X = OH) beispielsweise mit Thionylchlorid herstellen kann. Neben den Carbonsäurechloriden der Formel II (X = Cl) lassen sich auch weitere aktivierte Säurederivate der Formel II in an sich bekannter Weise direkt aus den zugrundeliegenden Benzoesäurederivaten (Formel II, X = OH) herstellen, wie beispielsweise die Methylester der Formel II mit X = OCH₃ durch Behandeln mit gasförmigem HCl in Methanol, die Imidazolide der Formel II durch Behandeln mit Carbonyldiimidazol (X = 1-Imidazolyl, Staab, Angew. Chem. Int. Ed. Engl. 1, 351 bis 367 (1962)), die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC). Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei ber Umsetzung der Benzoesäuremethylester (II, X = OMe) mit Guanidin Methanol oder THF zwischen 20°C und Siedetemperatur dieser Lösungsmittel bewährt. Bei den meisten Umsetzungen von Verbindungen II mit Guanidin wurde vorteilhaft in aprotischen inerten Lösungsmitteln wie THF, Dimethoxyethan, Dioxan gearbeitet. Aber auch Wasser kann als Lösungsmittel bei der Umsetzung von II und Guanidin verwendet werden.

Wenn X = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II sind bekannt und in der Literatur beschrieben. Die unbekannten Verbindungen der Formel II können nach literaturbekannten Methoden hergestellt werden, indem man beispielsweise 4-Chlor- oder 4-Fluor-3-chlor-sulfonylbenzoesäure mit Ammoniak oder einem Amin in eine 3-Aminosulfonyl-4-chlor- oder -fluorbenzoesäure (IIIa) bzw. mit einem schwachen Reduktionsmittel wie Natriumbisulfit und anschließender Alkylierung in einer 3-Alkylsulfonyl-4-chlor- oder -4-fluorbenzoesäure (IIIb, n = 2) 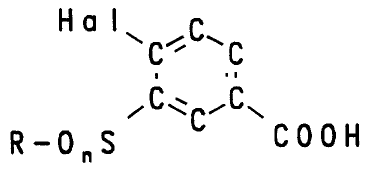
IIIa): R = R(3)
IIIb): R = NR(4)R(5)
überführt und die erhaltenen Benzoesäuren nach einer der oben beschriebenen Verfahrensvarianten zu erfindungsgemäßen Verbindungen I umgesetzt werden.

Verbindungen der Formeln IIIa) und b) können aber auch als Ausgangsverbindungen anderer Carbonsäuren dienen, wobei das Halogen in R(1)-Position sehr bequem gegen zahlreiche nucleophile Reagentien, wie Mercaptane R(3)-SH oder primäre Aminen R(4)R(5)NH unter Bildung weiterer Benzoesäurederivate II mit X = OH ausgetauscht werden kann.
In ähnlicher Weise können ausgehend von 3-Nitro-4-chlorbenzoesäure durch nucleophile Einführung eines erfindungsgemäßen Restes R(1) in Position 4 (Austausch gegen Cl) und weiterer Abwandlung der Nitrogruppe, wie Reduktion zu NH₂ und nachfolgende Alkylierung oder Verdrängung, beispielsweise durch Diazotierung und Sandmeyer-Reaktion, weitere Benzoesäurederivate (II, X = OH) hergestellt werden.

Benzoylguanidine I sind im allgemeinen schwache Basen und können Säure unter Bildung von Salzen binden. Als Säureadditionssalze kommen Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.
Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes des Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff überlicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg, vorzugsweise 0,01 mg bis höchstens 10 mg, vorzugsweise höchstens 1 mg. Bei akuten Ausbrüchen der Krankheit, etwa unmittelbar nach Erleiden des Herzinfarkts, können auch noch höhere und vor allem häufigere Dosierungen notwendig sein, z. B. bis zu 4 Einzeldosen pro Tag. Insbesondere bei i. v. Anwendung, etwa bei einem Infarktpatienten auf der Intensivstation können bis zu 100 mg pro Tag notwendig werden.

### Beispiele

### Allgemeine Vorschrift I zur Herstellung von Benzoyl-guanidinen (I) aus Benzoesäuren (II, X = OH)

0,01 M des Benzoesäurederivates der Formel II löst bzw. suspendiert man in 60 ml wasserfreiem Tetrahydrofuran (THF) und versetzt sodann mit 1,78 g (0,011 M) Carbonyldiimidazol. Nach dem Rühren über 2 Stunden bei Raumtemperatur werden 2,95 g (0,05 M) Guanidin in die Reaktionslösung eingetragen. Nach dem Rühren über Nacht destilliert man das THF unter vermindertem Druck (Rotationsverdampfer) ab, versetzt mit Wasser, stellt mit 2N HCl auf pH 6 - 7 und filtriert das entsprechende Benzoylguanidin (Formel I) ab.
Die so erhaltenen Benzoylguanidine können durch Behandeln mit wäßriger oder methanolischer Salzsäure oder anderen pharmakologisch verträglichen Säuren in die entsprechenden Salze überführt werden.

### Allgemeine Vorschrift II zur Herstellung von Benzoylguanidinen (I) aus Benzoesäureestern (allgem. Formel II, X = Alkoxy oder Phenoxy)

1 Äquivalent eines entsprechenden Benzoesäureesters der Formel II sowie 5.0 Äquivalente Guanidin werden in Isopropanol gelöst oder in THF suspendiert, und dann wird bis zur vollständigen Umsetzung (TLC-Kontrolle) am Rückflußkühler gekocht (Reaktionszeit etwa 2 - 5 Stunden). Das Lösungsmittel wird unter verminderten Druck (im Rotationsverdampfer) abdestilliert, der Rückstand wird in Ethylacetat aufgenommen und 3 mal mit gesättigter Natriumhydrogencarbonat-Lösung gewaschen. Trocknung der organischen Phase über Natriumsulfat, Abdestillieren des Lösungsmittels unter vermindertem Druck und Chromatographie des Rückstandes an Kieselgel unter Anwendung eines geeigneten Laufmittels.
Überführung des erhaltenen entsprechenden Benzoylguanidins der Formel I in das korrespondierende Hydrochlorid erfolgt analog Vorschrift I.

### Beispiel 1:

### 4-Ethylamino-3-methylsulfonylbenzoyl-guanidin-hydrochlorid, Schmp.: 242°C.

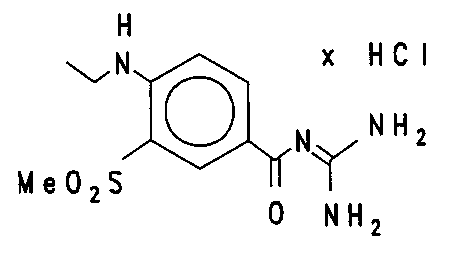

### Beispiel 2:

### 4-N,N-Diethylamino-3-methylsulfonylbenzoyl-guanidin-hydrochlorid, Schmp.: 274°C.

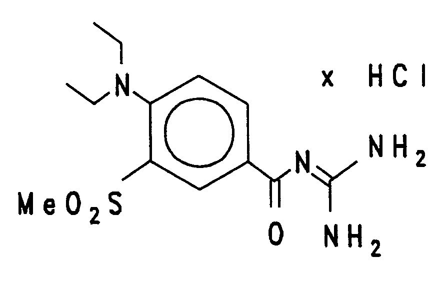

### Beispiel 3:

### 4-(4-Chlorphenoxy)-3-methylsulfonylbenzoyl-guanidin-hydrochlorid, Schmp.: 292-293°C.

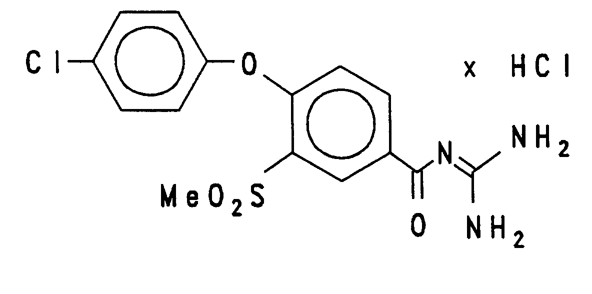

### Beispiel 4:

### 4-(4-Fluorphenoxy)-3-methylsulfonylbenzoyl-guanidin-hydrochlorid, Schmp.: 302-304°C

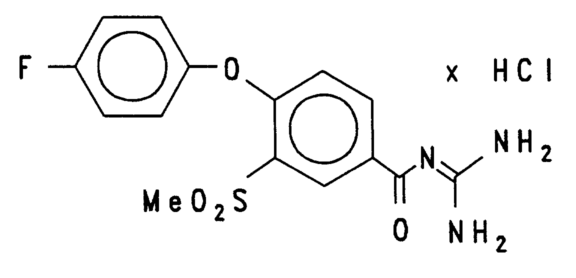

### Beispiel 5:

### 4-(4-Fluoranilino)-3-methylsulfonylbenzoyl-guanidin-hydrochlorid, Schmp.: 287°C.

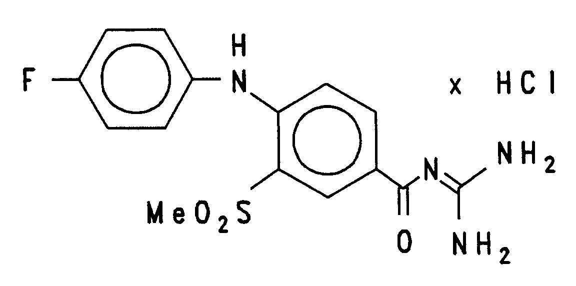

### Beispiel 6:

### 4-(3-Chlor-4-fluoranilino)-3-methylsulfonylbenzoyl-guanidin-hydrochlorid, Schmp.: 298-300°C.

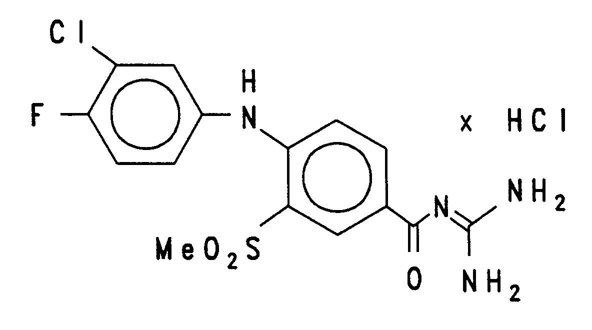

### Beispiel 7:

### 4-Isopropyl-3-methylsulfonylbenzoyl-guanidin-hydrochlorid, Schmp.: 268°C (Zers.).

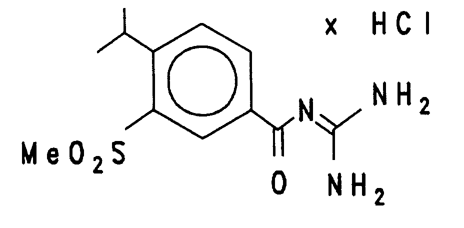

### Beispiel 8:

### 4-Isopropyl-3-methylsulfonylbenzoyl-guanidin-methansulfonat Schmp.: 226-228°C.

### Syntheseweg:

a)
   4-Isopropyl-3-chlorsulfonylbenzoesäure (Schmp. 203 - 204°C) durch Erhitzen von 4-Isopropylbenzoesäure mit Chlorsulfonsäure bei 95°C für 3 Stunden
b)
   2-Isopropyl-5-carboxybenzolsulfinsäure (Schmp. 205 - 207°C) aus a) durch Reduktion mit Natriumsulfit bei 60°C in wäßriger NaOH, bei pH 9 - 10.
c)
   4-Isopropyl-3-methylsulfonylbenzoesäure (Schmp. 209 - 211°C) aus b) durch Alkylierung mit Methylbromid in Gegenwart von NaOH in DMF bei 60°C für 3 Stunden.
d)
   4-Isopropyl-3-methylsulfonylbenzoyl-guanidin-methansulfonat aus c) durch Reaktion mit Thionylchlorid in Toluol (Rückfluß) für 1 Stunde. Nach Verdampfen des Lösungsmittels wird der Rückstand in THF gelöst und in ein Gemisch aus Guanidinhydrochlorid, 2N NaOH und THF gegeben. Nach 4 Stunden bei 30 - 40°C wird das THF abdestilliert und das kristalline 4-Isopropyl-3-methylsulfonylbenzoyl-guanidin abfiltriert. Zur Salzbildung schließt sich die Umsetzung mit Methansulfonsäure an.

### Beispiel 9:

### 4-Phenoxy-3-methylsulfonylbenzoyl-guanidin-hydrochlorid, Fp.: 300°C.

Herstellung der 4-Phenoxy-3-methylsulfonylbenzoesäure (Ausgangsmaterial, Fp 183 - 185°C) durch Umsetzung von 4-Chlor-3-methylsulfonylbenzoesäuremethylester mit Kaliumphenolat in siedendem N,N-Dimethylacetamid über 16 Stunden, Abdestillieren des Lösungsmittel, Auflösen in Wasser und Ansäuern mit wäßriger HCl auf pH 1.

Weitere Umsetzung gemäß Vorschrift I zur Titelverbindung.

### Beispiel 10:

### 3-Methylsulfonyl-4-(2,6-cis-dimethylpiperidino)benzoyl-guanidin-methansulfonat, Fp. 203°C.

### Herstellung der 3-Methylsulfonyl-4-(2,6-cis-dimethylpiperidino)-benzoesäure

(Ausgangsmaterial gemäß Vorschrift I; viskoses amorphes Produkt) durch Kochen von 4-Fluor-3-methylsulfonylbenzoesäure in überschüssigem 2,6-cis-Dimethylpiperidin über 24 Stunden, Abdestillieren des Lösungsmittels und anschließendem Ansäuern auf pH 1 mit Salzsäure.

### Beispiel 11:

### 4-(1-Methylpropyl)-3-methylsulfonylbenzoyl-guanidin-hydrochlorid Fp. 228 - 230°C.

### Syntheseweg:

a)
   4-(1-Methylpropyl)-3-methylsulfonylbenzoesäuremethylester wird erhalten aus 4-Brom-3-methylsulfonyl-benzoesäuremethylester durch cross-coupling mit 1,5 Äquivalenten Isobutylzink-chlorid (aus sec.-Butylmagnesiumchlorid durch Transmetallierung mit Zink(II)chlorid-Etherat in THF) durch Rühren bei Zimmertemperatur in Gegenwart von katalytischen Mengen von Palladium(II)[1,1'-bis-(diphenylphosphino)-ferrocen]chlorid und Kupfer-(I)iodid, wäßrige Aufarbeitung, Extraktion mit Essigester und anschließende Säulenchromatographie an Kieselgel mit Essigester/n-Heptan (3 : 7), farbloses Öl.
b)
   4-(1-Methylpropyl)-3-methylsulfonylbenzoyl-guanidin-hydrochlorid aus a) durch Kochen in THF in Gegenwart von Guanidin analog allgemeiner Vorschrift II und anschließender Behandlung mit HCl zur Bildung des Hydrochlorides.

### Beispiel 12:

### 4-Butyl-3-methylsulfonylbenzoyl-guanidin-hydrochlorid, Fp. 213-215 °C.

### Beispiel 13:

### 4-(2-Methylpropyl)-3-methylsulfonylbenzoyl-guanidin-hydrochlorid, Fp. 229 - 230°C.

### Syntheseweg:

a)
   4-(2-Methylpropyl)-3-methylsulfonylbenzoesäuremethylester wird erhalten aus 4-Brom-3-methylsulfonyl-benzoesäuremethylester durch cross-coupling mit 1,5 Äquivalenten Isobutylzink-chlorid (aus Isobutylmagnesiumchlorid durch Transmetallierung mit Zink(II)chlorid-Etherat in THF) durch Rühren bei Zimmertemperatur in Gegenwart von katalytischen Mengen von Palladium(II)[1,1'-bis-(diphenylphosphino)-ferrocen]chlorid und Kupfer-(I)iodid, wäßrige Aufarbeitung, Extraktion mit Essigester und anschließende Säulenchromatographie an Kieselgel mit Essigester/n-Heptan (3 : 7), farbloses Öl.
b)
   4-(2-Methylpropyl)-3-methylsulfonylbenzoyl-guanidin-hydrochlorid aus a) durch Kochen in THF in Gegenwart von Guanidin analog allgemeiner Vorschrift II und anschließender Behandlung mit HCl zur Bildung des Hydrochlorides.

### Beispiel 14:

### 4-(4-Hydroxyphenoxy)-3-methylsulfonylbenzoyl-guanidin-hydrochlorid, Fp. 258 - 259°C;

Herstellung durch katalytische Hydrierung von 4-(4-Benzyloxyphenoxy)-3-methylsulfonylbenzoyl-guanidin-hydrochlorid (Beispiel 78) mit Pd/C-Katalysator in Eisessig bei Zimmertemperatur und 760 mm Druck.

## Patentansprüche

1. Benzoylguanidin, ausgewählt aus der Gruppe, bestehend aus 4-Ethylamino-3-methylsulfonylbenzoyl- guanidin-hydrochlorid, 4-N,N-Diethylamino-3-methylsulfonylbenzoyl-guanidin- hydrochlorid, 4-(4-Chlorphenoxy)-3-methylsulfonylbenzoyl-guanidin-hydrochlorid, 4-(3-Chlor-4-fluorphenoxy)-3-methylsulfonylbenzoyl-guanidin-hydrochlorid, 4-(4-Fluorphenoxy)-3-methylsulfonylbenzoyl-guanidin-hydrochlorid, 4-(4-Fluoranilino)-3-methylsulfonylbenzoyl-guanidin-hydrochlorid, 4-(3-Chlor-4-fluoranilino)-3-methylsulfonylbenzoyl-guanidin-hydrochlorid, 4-Isopropyl-3-methylsulfonylbenzoyl-guanidin-hydrochlorid, 4-Isopropyl-3-methylsulfonylbenzoyl-guanidin-methansulfonat, 4-Phenoxy-3-methylsulfonylbenzoyl-guanidin-hydrochlorid, 3-Methylsulfonyl-4-(2,6-cis-dimethylpiperidino)benzoyl-guanidin-methansulfonat, 4-(1-Methylpropyl)-3-methylsulfonylbenzoyl-guanidin-hydrochlorid, 4-Butyl-3-methylsulfonylbenzoyl-guanidin-hydrochlorid, 4-(2-Methylpropyl)-3-methylsulfonylbenzoyl-guanidin-hydrochlorid und 4-(4-Hydroxyphenoxy)-3-methylsulfonylbenzoyl-guanidin-hydrochlorid.

2. Verbindung nach Anspruch 1, dadurch gekennzeichnet, daß sie ausgewählt ist aus der Gruppe, bestehend aus 4-Isopropyl-3-methylsulfonylbenzoyl-guanidin-methansulfonat, 4-Isopropyl-3-methylsulfonylbenzoyl-guanidin-hydrochlorid, 4-Phenoxy-3-methylsulfonylbenzoyl-guanidin-hydrochlorid und 4-(4-Chlorphenoxy)-3-methylsulfonylbenzoyl-guanidin-hydrochlorid, sowie deren weiteren pharmakologisch verträglichen Salze.

3. Verbindung I nach Anspruch 1, dadurch gekennzeichnet, daß sie 4-Isopropyl-3-methylsulfonylbenzoyl-guanidin-methansulfonat ist.

4. Verfahren zur Herstellung einer Verbindung I nach Anspruch 1, dadurch gekennzeichnet, daß man
eine Verbindung der Formel II 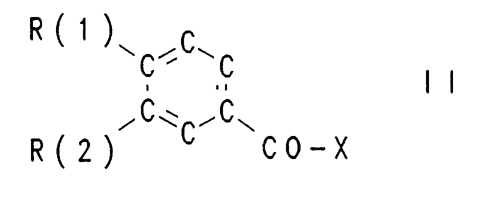 worin R(1) und R(2) die dem im Endprodukt entsprechende Bedeutung hat, und worin X für eine leicht nucleophil substituierbare leaving group steht,
mit Guanidin umsetzt.

5. Verwendung einer Verbindung I nach Anspruch 1 zur Herstellung eines Medikaments zur Behandlung von Arrhythmien.

6. Verwendung einer Verbindung I nach Anspruch 1 zur Herstellung eines Medikaments zur Behandlung oder Prophylaxe des Herzinfarktes.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung I nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch I zur Herstellung eines Medikaments zur Behandlung von Krankheiten, bei denen die Zellproliferation eine primäre oder sekundäre Ursache darstellt, und somit ihre Verwendung als Antiatherosklerotika, Mittel gegen Diabetische Spätkomplikationen, Krebserkrankungen, fibrotische Erkrankungen wie Lungenfibrose, Leberfibrose oder Nierenfibrose, Prostatahyperplasie.

15. Verwendung einer Verbindung I nach Anspruch 1 zur Herstellung eines wissenschaftliches Tools zur Inhibition des Na⁺/H⁺-Exchangers, zur Diagnose der Hypertonie und proliferativer Erkrankungen.

## Claims

1. A benzoylguanidine selected from the group consisting of 4-ethylamino-3-methylsulfonylbenzoylguanidine hydrochloride, 4-N,N-diethylamino-3-methylsulfonylbenzoylguanidine hydrochloride, 4-(4-chlorophenoxy)-3-methylsulfonylbenzoylguanidine hydrochloride, 4-(3-chloro-4-fluorophenoxy)-3-methylsulfonylbenzoylguanidine hydrochloride, 4-(4-fluorophenoxy)-3-methylsulfonylbenzoylguanidine hydrochloride, 4-(4-fluoroanilino)-3-methylsulfonylbenzoylguanidine hydrochloride, 4-(3-chloro-4-fluoroanilino)-3-methylsulfonylbenzoylguanidine hydrochloride, 4-isopropyl-3-methylsulfonylbenzoylguanidine hydrochloride, 4-isopropyl-3-methylsulfonylbenzoylguanidine methanesulfonate, 4-phenoxy-3- methylsulfonylbenzoylguanidine hydrochloride, 3-methylsulfonyl-4-(2,6-cis-dimethylpiperidino)benzoylguanidine methanesulfonate, 4-(1-methylpropyl)-3-methylsulfonylbenzoylguanidine hydrochloride, 4-butyl-3-methylsulfonylbenzoylguanidine hydrochloride, 4-(2-methylpropyl)-3-methylsulfonylbenzoylguanidine hydrochloride and 4-(4-hydroxyphenoxy)-3-methylsulfonylbenzoylguanidine hydrochloride.

2. A compound as claimed in claim 1, which is selected from the group consisting of 4-isopropyl-3-methylsulfonylbenzoylguanidine methanesulfonate, 4-isopropyl-3-methylsulfonylbenzoylguanidine hydrochloride, 4-phenoxy-3-methylsulfonylbenzoylguanidine hydrochloride and 4-(4-chlorophenoxy)-3-methylsulfonylbenzoylguanidine hydrochloride, and their other pharmacologically tolerable salts.

3. A compound I as claimed in claim 1, which is 4-isopropyl-3-methylsulfonylbenzoylguanidine methane-sulfonate.

4. A process for the preparation of a compound I as claimed in claim 1, which comprises reacting a compound of the formula II 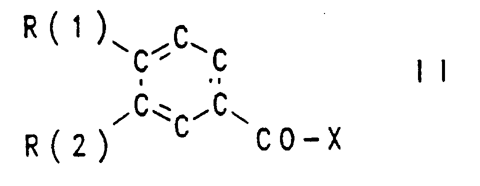 in which R(1) and R(2) have the meaning corresponding to the final product, and in which X is a leaving group which can be easily substituted nucleophilically, with guanidine.

5. The use of a compound I as claimed in claim 1 for the production of a medicament for the treatment of arrhythmias.

6. The use of a compound I as claimed in claim 1 for the production of a medicament for the treatment or prophylaxis of cardiac infarct.

7. The use of a compound I as claimed in claim 1 for the production of a medicament for the treatment or prophylaxis of angina pectoris.

8. The use of a compound I as claimed in claim 1 for the production of a medicament for the treatment or prophylaxis of ischemic conditions of the heart.

9. The use of a compound I as claimed in claim 1 for the production of a medicament for the treatment or prophylaxis of ischemic conditions of the peripheral and central nervous system and of stroke.

10. The use of a compound I as claimed in claim 1 for the production of a medicament for the treatment or prophylaxis of ischemic conditions of peripheral organs and limbs.

11. The use of a compound I as claimed in claim 1 for the production of a medicament for the treatment of conditions of shock.

12. The use of a compound I as claimed in claim 1 for the production of a medicament for use in surgical operations and organ transplants.

13. The use of a compound I as claimed in claim 1 for the production of a medicament for the preservation and storage of transplants for surgical measures.

14. The use of a compound I as claimed in claim 1 for the production of a medicament for the treatment of diseases in which cell proliferation is a primary or secondary cause, and thus their use as antiatherosclerotics, agents against diabetic late complications, cancers, fibrotic diseases such as pulmonary fibrosis, fibrosis of the liver or fibrosis of the kidney, and prostate hyperplasia.

15. The use of a compound I as claimed in claim 1 for the production of a scientific tool for the inhibition of the Na⁺/H⁺ exchanger, for the diagnosis of hypertension and proliferative diseases.

## Revendications

1. Benzoylguanidine choisie parmi le groupe constitué du chlorhydrate de 4-éthylamino-3-méthylsulfonylbenzoyl-guanidine, du chlorhydrate de 4-N,N-diéthylamino-3-méthylsulfonylbenzoyl-guanidine, du chlorhydrate de 4-(4-chlorophénoxy)-3-methylsulfonylbenzoyl-guanidine, du chlorhydrate de 4-(3-chloro-4-fluorophénoxy)-3-méthylsulfonylbenzoylguanidine, du chlorhydrate de 4-(4-fluorophénoxy)-3-méthylsulfonylbenzoyl-guanidine, du chlorhydrate de 4-(4-fluoroanilino)-3-méthylsulfonylbenzoyl-guanidine, du chlorhydrate de 4-(3-chloro-4-fluoroanilino)-3-méthylsulfonylbenzoyl-guanidine, du chlorhydrate de 4-isopropopyl-3-méthylsulfonylbenzoyl-guanidine, du méthanesulfonate de 4-isopropopyl-3-méthylsulfonylbenzoylguanidine, du chlorhydrate de 4-phénoxy-3-méthylsulfonylbenzoyl-guanidine, du méthanesulfonate de 3-méthylsulfonyl-4-(2,6-cis-diméthylpipéridino)-benzoylguanidine,
du chlorhydrate de 4-(1-méthylpropyl)-3-méthylsulfonylbenzoyl-guanidine, du chlorhydrate de 4-butyl-3-méthylsulfonylbenzoyl-guanidine, du chlorhydrate de 4-(2-méthylpropyl)-3-méthylsulfonylbenzoyl-guanidine, et du chlorhydrate de 4-(4-hydroxyphénoxy)-3-méthylsulfonylbenzoyl-guanidine.

2. Composé selon la revendication 1, caractérisé en ce qu'il est choisi parmi le groupe constitué du méthanesulfonate de 4-isopropopyl-3-méthylsulfonylbenzoylguanidine, du chlorhydrate de 4-isopropopyl-3-méthylsulfonylbenzoyl-guanidine, du chlorhydrate de 4-phénoxy-3-méthylsulfonylbenzoyl-guanidine, du chlorhydrate de 4-(4-chlorophénoxy)-3-méthylsulfonylbenzoyl-guanidine.

3. Composé selon la revendication 1, caractérisé en ce qu'il est le méthanesulfonate de 4-isopropopyl-3-méthylsulfonyl-benzoyl-guanidine.

4. Procédé de fabrication d'un composé selon la revendication 1, caractérisé en ce qu'on fait réagir un composé de Formule II 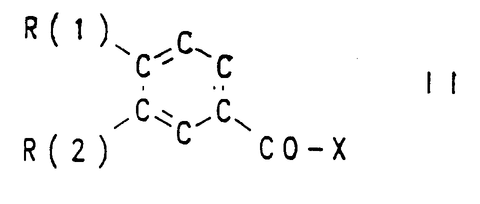 dans laquelle R(1) et R(2) ont la signification correspondant au produit final et dans laquelle X représente un groupe partant facilement substituable par un nucléophile, avec la guanidine.

5. Utilisation d'un composé I selon la revendication 1 pour la fabrication d'un médicament pour le traitement des arythmies.

6. Utilisation d'un composé I selon la revendication 1 pour la fabrication d'un médicament pour le traitement ou la prophylaxie des infarctus cardiaques.

7. Utilisation d'un composé I selon la revendication 1 pour la fabrication d'un médicament pour le traitement ou la prophylaxie de l'angine de poitrine.

8. Utilisation d'un composé I selon la revendication 1 pour la fabrication d'un médicament pour le traitement ou la prophylaxie des états ischémiques du coeur.

9. Utilisation d'un composé I selon la revendication 1 pour la fabrication d'un médicament pour le traitement ou la prophylaxie des états ischémiques du système nerveux périphérique et central et de l'apoplexie.

10. Utilisation d'un composé I selon la revendication 1 pour la fabrication d'un médicament pour le traitement ou la prophylaxie des états ischémiques des organes périphériques et des membres.

11. Utilisation d'un composé I selon la revendication 1 pour la fabrication d'un médicament pour le traitement des états de choc.

12. Utilisation d'un composé I selon la revendication 1 pour la fabrication d'un médicament à utiliser au cours des opérations chirurgicales et des transplantations d'organes.

13. Utilisation d'un composé I selon la revendication 1 pour la fabrication d'un médicament pour la conservation et le stockage des transplants à des fins chirurgicales.

14. Utilisation d'un composé I selon la revendication 1 pour la fabrication d'un médicament pour le traitement de maladies dans lesquelles la prolifération cellulaire représente-une cause primaire ou secondaire, et par conséquent son utilisation comme antiathérosclérotique, comme agent contre les complications tardives du diabète, les maladies cancéreuses, les maladies fibrotiques comme la fibrose des poumons, la fibrose du foie et la fibrose des reins, l'hyperplasie de la prostate.

15. Utilisation d'un composé I selon la revendication 1 pour la fabrication d'un outil efficace pour l'inhibition des échangeurs Na⁺/H⁺, pour le diagnostique de l'hypertonie et des maladies prolifératives.
